Europäisches Patentamt

European Patent Office

Office européen des brevets

(19)

(11) Numéro de publication : **0 398 813 B1**

(12) **FASCICULE DE BREVET EUROPEEN**

(45) Date de publication du fascicule du brevet :
28.07.93 Bulletin 93/30

(51) Int. Cl.$^5$ : **A61F 2/16**

(21) Numéro de dépôt : **90401323.2**

(22) Date de dépôt : **18.05.90**

(54) **Composant optique, tel qu'implant intra-oculaire ou lentille de contact, propre à la correction de la vision d'un individu.**

(30) Priorité : **19.05.89 FR 8906594**

(43) Date de publication de la demande :
**22.11.90 Bulletin 90/47**

(45) Mention de la délivrance du brevet :
**28.07.93 Bulletin 93/30**

(84) Etats contractants désignés :
**CH DE ES GB IT LI NL**

(56) Documents cités :
**US-A- 4 469 413**
**US-A- 4 504 982**
**US-A- 4 710 193**

(56) Documents cités :
**JOURNAL OF THE OPTICAL SOCIETY OF AMERICA, vol. 2, no. 8, août 1985, pages1273-1281, Optical Society of America, New York, US; R. NAVARRO et al.:"Accommodation-dependent model of the human eye with aspherics"**

(73) Titulaire : **ESSILOR INTERNATIONAL, Cie Générale d'Optique**
**1 Rue Thomas Edison, Echat 902**
**F-94028 Créteil Cédex (FR)**

(72) Inventeur : **Mercier, Jean-Louis**
**3 Rue du Bon Puits**
**F-91640 Fontenay Les Briis (FR)**

(74) Mandataire : **CABINET BONNET-THIRION**
**95 Boulevard Beaumarchais**
**F-75003 Paris (FR)**

Jouve, 18, rue Saint-Denis, 75001 PARIS

## Description

La présente invention concerne d'une manière générale les composants optiques mis en oeuvre pour la correction de la vision d'un individu.

Elle vise plus particulièrement le cas des implants intra-oculaires, tout en pouvant aussi bien s'étendre aux lentilles de contact.

Ainsi qu'on le sait, un implant intra-oculaire est destiné à être substitué à un cristallin défaillant.

Le plus souvent, un tel implant intra-oculaire n'est pas accommodatif.

C'est le cas, par exemple, pour celui faisant l'objet du brevet américain US-A-4.504.982.

En effet, si, dans ce brevet américain, la partie centrale de la face avant de l'implant intra-oculaire concerné forme une surface asphérique de révolution de méridien répondant à la formule suivante :

$$X = \frac{1}{R}\left[\frac{Y^2}{1 + \sqrt{1 - (1+K)\ Y^2/R^2}}\right] + A2\ Y^4 + A3\ Y^6 + A4\ Y^8 + A5\ Y^{10}$$

dans laquelle R, K, A2, A3, A4 et A5 sont des paramètres numériques, cet implant intra-oculaire, pris dans son ensemble, est de puissance constante, les paramètres numériques en question étant simplement choisis de manière à ce que la plus grande partie de son aberration sphérique longitudinale soit corrigée.

De même, l'implant intra-oculaire faisant l'objet du brevet américain US-A-4.769.033 n'est pas accommodatif, dans la mesure où, bifocal, il ne permet pas une vision intermédiaire entre la vision de loin et la vision de près.

De tels implants intra-oculaires non accommodatifs ont pour inconvénient évident de ne pas satisfaire par eux-mêmes à tous les cas de vision, et, donc, s'agissant notamment de l'implant intra-oculaire à puissance constante, de nécessiter parfois le port de lunettes.

Un implant intra-oculaire accommodatif se trouve cependant proposé dans le brevet américain US-A-4.710.193.

Mais, diffractif, cet implant intra-oculaire conduit en pratique à des aberrations chromatiques non négligeables.

La présente invention a pour objet un composant optique, en particulier un implant intra-oculaire, accommodatif et avantageusement exempt de cet inconvénient.

Pour ce faire, et alors que, à ce jour, le calcul des implants intra-oculaires est pour l'essentiel établi sur la base de la seule puissance qui doit être la leur, en les isolant en tant que tels dans l'air, sans se préoccuper de l'aberration sphérique longitudinale à laquelle ils conduisent dans l'oeil, le parti est pris, au contraire, suivant l'invention, de considérer qu'une fois en place un tel implant intra-oculaire forme l'un des constituants d'un ensemble optique dont les autres constituants sont ceux de l'oeil concerné, et de donner à cet implant intra-oculaire les formes de surface nécessaires pour que, dans cet ensemble optique, ils conduisent à une aberration sphérique longitudinale donnée, dûment estimée par le calcul.

Le problème, en l'espèce, est que les constituants d'un oeil, et donc leurs caractéristiques, varient d'un individu à un autre.

Le parti est donc également pris, suivant l'invention, de s'en référer à un oeil théorique donné, choisi comme standard.

Il s'agit, préférentiellement, de l'oeil théorique décrit dans l'article de R. NAVARRO et autres, paru, sous le titre "Accommodation-dependent model of the human eye with aspherics", dans la revue "J. Opt. Soc. Am. A." vol. 2, No 8, d'août 1985.

Mais, en variante, un autre oeil théorique pourrait tout aussi bien être choisi.

Cela étant, l'implant intra-oculaire, ou, d'une manière plus générale, le composant optique suivant l'invention, est du genre comportant deux faces, l'une avant, l'autre arrière, dont l'une au moins forme, dans sa partie centrale au moins, une surface asphérique de révolution de méridien répondant à la formule suivante :

$$X = \frac{1}{R_1} \left[ \frac{Y^2}{1 + \sqrt{1 - (1+K) \; Y^2/R_1^2}} \right] + A2 \; Y^4 + A3 \; Y^6 + A4 \; Y^8 + A5 \; Y^{10}$$

dans laquelle R1, K, A2, A3, A4 et A5 sont des paramètres numériques, et il est d'une manière générale caractérisé en ce que lesdits paramètres numériques sont choisis de manière telle que, pour l'ensemble optique constitué, d'une part, d'un oeil théorique donné, dont on a enlevé le cristallin si le composant optique concerné est un implant intra-oculaire, et, d'autre part, dudit composant optique, ils conduisent, pour la proximité objet P, définie par la formule :

$$P = N' \cdot \frac{dx'}{f'^2}$$

dans laquelle N' est l'indice du milieu image, dx' l'aberration sphérique longitudinale dans l'espace image, et f' la focale de l'oeil théorique choisi, à une courbe représentative,

qui, (I), pour les valeurs fortes de la hauteur H des rayons lumineux par rapport à l'axe, comporte un premier tronçon sensiblement droit de pente au plus égale à zéro et tout entier situé entre une droite verticale passant par une origine de référence quelconque donnée et une droite oblique passant par des points P1, P2 de coordonnées (-1 ; 1,5) et (-1,5 ; 2,75) par rapport à ladite origine de référence,

qui, (II), pour les valeurs faibles de la hauteur H des rayons lumineux par rapport à l'axe, comporte un deuxième tronçon recoupant verticalement l'axe des dioptries entre des points P3, P4 de cet axe d'abscisses (-4) et (-2,5) par rapport à l'origine de référence,

et qui, (III), pour les valeurs moyennes de la hauteur H des rayons lumineux par rapport à l'axe, comporte un tronçon médian se raccordant de manière monotone et continue aux deux tronçons précédents.

Ainsi, suivant l'invention, et pour le meilleur confort de l'usager, il est obtenu, pour l'ensemble optique constitué du composant optique suivant l'invention et de l'oeil auquel il est appliqué, des caractéristiques de caustique à deux pointes conduisant chacune respectivement à des conditions de vision correctes en vision de loin et en vision de près, avec, dans l'intervalle, une possibilité de vision intermédiaire.

Comme recherché, et s'agissant plus particulièrement d'un implant intra-oculaire substitué à un cristallin défaillant, il est ainsi procédé, suivant l'invention, à une réelle restitution de la capacité d'accommodation perdue par l'oeil, tout en maintenant dans les limites qu'il accepte naturellement l'aberration sphérique longitudinale.

Suivant une première forme de mise en oeuvre possible, cette aberration sphérique longitudinale est, pour la vision de loin, celle de l'oeil.

Suivant une deuxième forme de mise en oeuvre possible, elle est dûment corrigée.

Dans ce deuxième cas, la proximité se trouve ainsi avantageusement dûment stabilisée en vision de loin.

Les possibilités de l'invention, ses caractéristiques et ses avantages, ressortiront d'ailleurs de la description qui va suivre, à titre d'exemple, en référence aux dessins schématiques annexés sur lesquels :

La figure 1 est une vue en coupe axiale d'un oeil équipé d'un composant optique suivant l'invention ;

la figure 2 est, à échelle supérieure, une vue en plan de ce composant optique ;

la figure 3 en est une vue en coupe axiale, suivant la ligne III-III de la figure 2 ;

la figure 4 est une vue partielle en coupe axiale de l'oeil théorique pris en considération suivant l'invention ;

La figure 5 est un diagramme se rapportant à la courbe de proximité objet de cet oeil théorique, pris isolément ;

la figure 6 est un diagramme qui, analogue à celui de la figure 5, se rapporte à la courbe de proximité objet de l'ensemble optique constitué de cet oeil théorique et du composant optique suivant l'invention ;

les figures 7 à 18 sont des diagrammes analogues à celui de la figure 6, pour, chacun respectivement, d'autres composants optiques suivant l'invention.

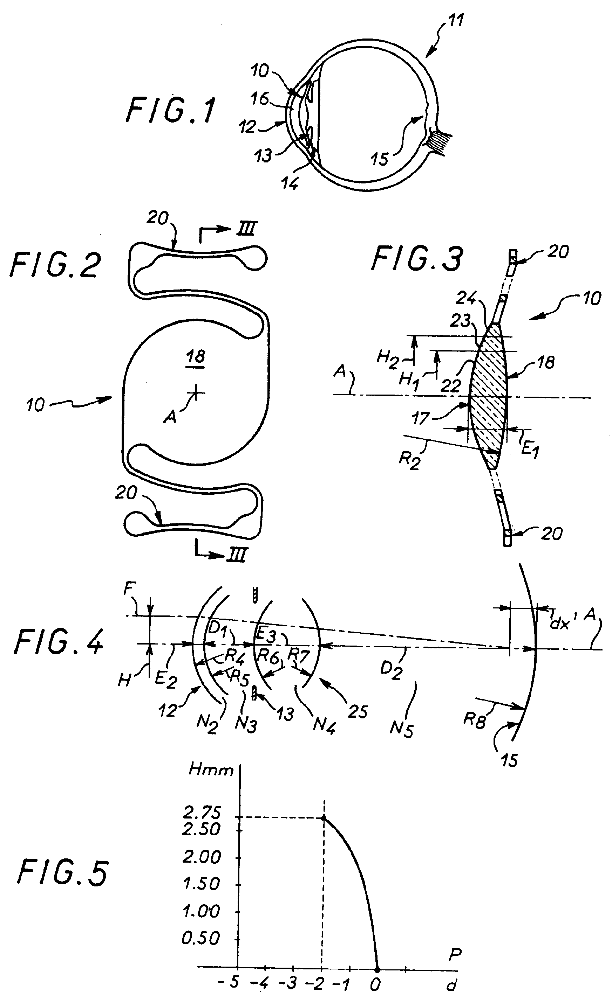

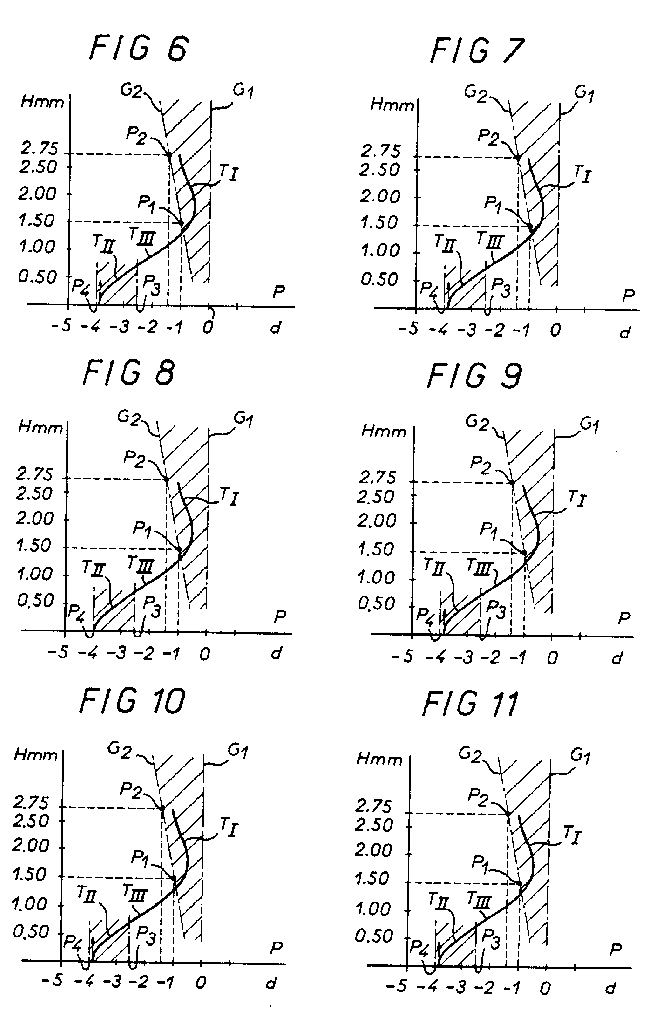

Les figures 1 à 3, et le diagramme de la figure 6 qui s'y rapporte, illustrent à titre d'exemple l'application de l'invention à la mise en oeuvre d'un implant intra-oculaire 10 en substitution au cristallin de l'oeil 11 à traiter.

On reconnaît, sur la figure 1, en 12 la cornée de cet oeil 11, en 13 son iris, en 14 le sac ayant initialement contenu son cristallin, et en 15 sa rétine.

Dans la forme de mise en oeuvre représentée, qui est une forme de mise en oeuvre préférentielle, l'implant intra-oculaire 10 suivant l'invention est implanté dans la chambre antérieure 16 de l'oeil 11, c'est-à-dire dans la partie de celui-ci qui s'étend entre sa cornée 12 et son iris 13.

De manière connue en soi, cet implant intra-oculaire 10 comporte une face avant 17 et une face arrière 18, et il est équipé, à sa périphérie, en positions diamétralement opposées l'un par rapport à l'autre, de deux bras élastiquement déformables 20, qui, chacun d'allure en S, sont propres à son appui sur les corps ciliaires de l'oeil 11, à la racine de l'iris 13 de celui-ci.

Les dispositions correspondantes étant bien connues par elles-mêmes, et ne faisant pas partie en soi de la présente invention, elles ne seront pas décrites plus en détail ici.

Elles sont d'ailleurs susceptibles de variantes, notamment en ce qui concerne la configuration et/ou le nombre des bras 20.

De manière également connue en soi, l'implant intra-oculaire 10 ainsi constitué peut par exemple être réalisé en matière synthétique, et par exemple en un polymère de métachrylate de méthyle.

Soit N1 l'indice de réfraction de son matériau constitutif, et soit A son axe.

Dans la forme de réalisation représentée sur les figures 1 à 3, l'implant intra-oculaire 10 suivant l'invention est bi-convexe.

Sa face avante 17 forme, dans sa partie centrale, une surface asphérique de révolution 22 de méridien répondant à la formule suivant :

$$X = \frac{1}{R_1} \left[ \frac{Y^2}{1 + \sqrt{1 - (1+K)\ Y^2/R_1^2}} \right] + A2\ Y^4 + A3\ Y^6 + A4\ Y^8 + A5\ Y^{10}$$

dans laquelle R1, K et A2, A3, A4 et A5 sont des paramètres numériques.

En pratique, R1 correspond au rayon de courbure d'une sphère de base, K à une constante de conicité, et A2, A3, A4 et A5 sont des paramètres d'asphéricité qui seront précisés ultérieurement.

En pratique, également, l'étendue de la surface asphérique 22 est limitée à une circonférence de rayon H1 au plus égale à 2,35 mm.

Au-delà, la partie périphérique de la face avante 17 comporte une surface torique 23 qui se raccorde tangentiellement à la surface asphérique 22, son rayon de courbure étant égal à celui de cette dernière à son raccordement à celle-ci.

Soit R3 ce rayon de courbure, et soit X3, Y3 les coordonnées du centre correspondant.

Cette surface torique 23 minimise avantageusement les conséquences d'un éventuel excentrement et/ou d'une valeur relativement grande, supérieure à 5,5 mm, pour le diamètre de la pupille d'entrée.

Elle permet également avantageusement de maîtriser les déformations en dehors de la surface centrale asphérique 22.

En pratique, l'étendue de cette surface torique 23 est elle-même limitée à une circonférence de rayon H2 de l'ordre de 3 mm.

Au-delà, l'implant intra-oculaire 10 forme un simple bourrelet annulaire 24 dont sont issus ses bras 20.

Dans la forme de réalisation représentée sur les figures 1 à 3, la face arrière 18 de cet implant intra-oculaire 10 est sphérique.

Soit R2 son rayon.

Soit E1, enfin, son épaisseur sur son axe A.

Pour le calcul d'un implant intra-oculaire 10 suivant l'invention, c'est-à-dire pour l'établissement de ses paramètres R1, K, A2, A3, A4 et A5, il est pris en considération un oeil théorique déterminé.

Comme indiqué ci-dessus, il s'agit, préférentiellement, de l'oeil théorique de R. NAVARRO et autres.

Cet oeil théorique est représenté schématiquement à la figure 4, sur laquelle la référence 12 désigne, comme précédemment, la cornée, la référence 13 l'iris, et la référence 15 la rétine.

Sur cette figure 4 est schématisé, en plus, en 25, le cristallin.

A toutes fins utiles, on rapportera ci-après, en mm, les caractéristiques dimensionnelles de cet oeil théorique, tel que précisé dans l'article mentionné ci-dessus :

- rayon R4 de la face avant de la cornée 12 : 7,72 avec K = -0,26
- rayon R5 de la face arrière de la cornée 12 : 6,5
- épaisseur E2 de la cornée 12 : O,55
- distance D1 de la face arrière de la cornée 12 à la face avant du cristallin 25 : 3,05
- rayon R6 de la face avant du cristallin 25 : 10,2 avec K = -3,1316
- rayon R7 de la face arrière du cristallin 25 : -6 avec K = -1
- épaisseur E3 du cristallin 25 : 4
- distance D2 de la face arrière du cristallin 25 à la rétine 15 : 16,341
- rayon R8 de la rétine 15 : -12

- indice N2 de la cornée 12 : 1,367
- indice N3 de l'humeur entre la cornée 12 et le cristallin 25 : 1,337
- indice N4 du cristallin 25 : 1,42
- indice N5 de l'humeur entre le cristallin 25 et la rétine 15 : 1,336.

Sur le diagramme de la figure 5 on a reporté, en abscisses, la proximité objet P de cet oeil théorique, en dioptries d, et, en ordonnées, la hauteur H par rapport à son axe, en mm.

En l'absence de toute aberration sphérique longitudinale, un rayon lumineux F parallèle à l'axe A et de hauteur H par rapport à celui-ci recoupe cet axe A sur la rétine 15.

Mais, en pratique, et tel que schématisé sur la figure 4, il existe, dans l'espace image correspondant, une aberration sphérique longitudinale dx'.

La proximité objet P ici prise en considération est définie par la formule :

$$P = \frac{N' \, dx'}{f'^2}$$

dans laquelle N' est l'indice de réfraction du milieu image et f' est la focale de l'oeil théorique.

En pratique, N' = 1,336.

Par mesure de commodité, la focale f' sera ici supposée constante, les effets de sa variation étant en effet en pratique négligeables.

Cette focale f' est par exemple comprise entre 18 et 25 mm.

Préférentiellement, elle est choisie arbitrairement égale à 21,5 mm.

Sur les bases de ce qui précède, un programme de tracé de rayons permet d'établir point par point la courbe représentative de la proximité objet P.

Il est procédé de même pour l'ensemble optique qui, à la manière de ce qui est représenté pour un oeil particulier à la figure 1, est constitué, d'une part, d'un oeil théorique donné, tel que celui représenté à la figure 4, dont on a enlevé le cristallin si, comme en l'espèce, le composant optique concerné est un implant intra-oculaire 10, et, d'autre part, de ce composant optique.

Mais, suivant l'invention, et tel que représenté sur le diagramme de la figure 6 pour l'implant intra-oculaire 10 représenté sur les figures 1 à 3, les paramètres numériques R1, K, A2, A3, A4 et A5 sont alors choisis de manière telle que, pour cet ensemble optique, ils conduisent, pour la proximité objet P, telle que définie ci-dessus, à une courbe représentantive

qui, (I), pour les fortes valeurs de la hauteur H des rayons lumineux par rapport à l'axe A, comporte un premier tronçon TI sensiblement droit de pente au plus égale à zéro et tout entier situé entre une droite verticale G1 passant par une origine de référence O quelconque donnée et une droite oblique G2 passant par des points P1, P2 de coordonnées, respectivement, (-1 ; 1,5) et (-1,5 ; 2,75) par rapport à cette origine de référence O,

qui, (II), pour les valeurs faibles de la hauteur H des rayons lumineux par rapport à l'axe, comporte un deuxième tronçon TII recoupant verticalement l'axe des dioptries entre des points P3, P4 de cet axe d'abscisses, respectivement, (-2,5) et (-4) par rapport à l'origine de référence O,

et qui, (III), pour les valeurs moyennes de la hauteur H des rayons lumineux par rapport à l'axe, comporte un tronçon médian TIII se raccordant de manière monotone et continue aux deux tronçons TI, TII précédents.

Pour faciliter la compréhension de l'invention, les secteurs dans lesquels doivent se situer les tronçons TI et TII de la courbe de proximité objet P ont été hachurés sur la figure 6.

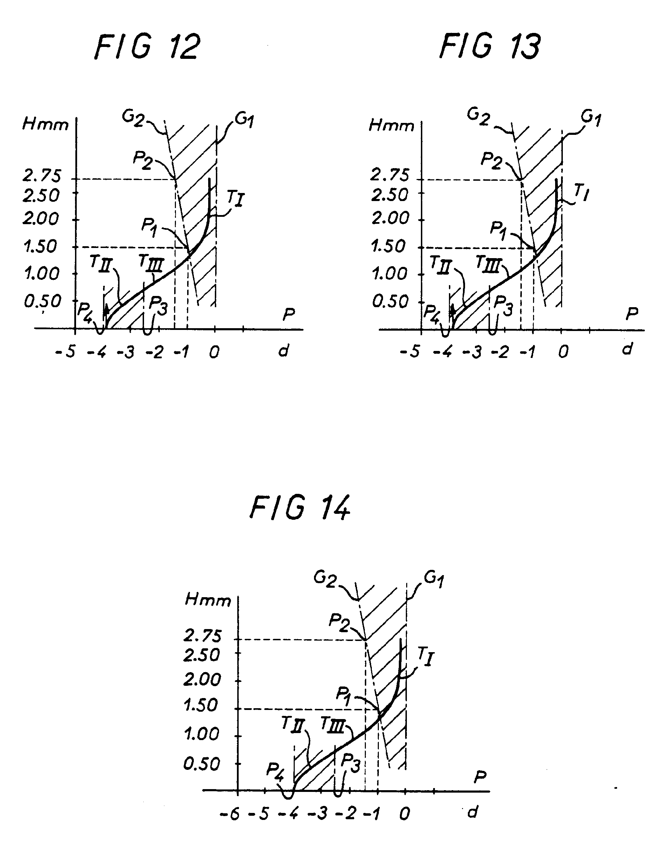

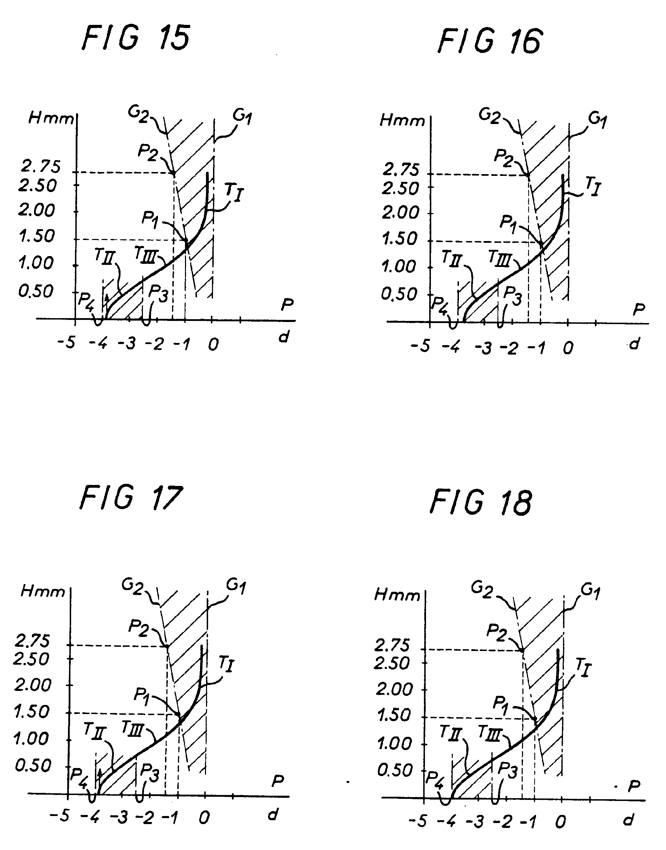

Il en est de même sur les figures 7 à 18.

Le tronçon TI correspond à la vision de loin, et le tronçon TII à la vision de près.

En pratique, suivant la profondeur, sur l'axe, de l'oeil 11 à traiter, et/ou le rayon de courbure de sa cornée, on ajuste en conséquence la vision de loin, par une translation de la courbe de proximité objet P le long de l'axe des dioptries, c'est-à-dire par une addition convenable de dioptries par rapport à l'origine de référence O choisie.

Il suffit, pour ce faire, d'ajuster en conséquence le rayon de l'une des surfaces sphériques en cause.

Cela peut être celui de la surface sphérique constituant la face arrière 18.

Mais cela peut aussi être le rayon R1 de la sphère de base de la surface asphérique 22 de la face avant 17.

Dans la forme de mise en oeuvre illustrée par la figure 6, les paramètres R1, K, A2, A3, A4 et A5 déterminant par ailleurs cette surface asphérique 22 sont en pratique choisis de manière telle que, en vision de loin, tronçon T1, l'aberration sphérique longitudinale due à l'ensemble optique constitué de l'oeil théorique pris en considération, cristallin en moins puisque le composant optique concerné est un implant intra-oculaire 10, et de ce composant optique, soit sensiblement la même que celle due au seul oeil théorique avec son cristallin.

Autrement dit, le tronçon T1 de la courbe de proximité objet P résultante a une allure sensiblement analogue à celle de la partie correspondante de la courbe de proximité objet P de l'oeil théorique pris en considération.

Quoi qu'il en soit, la courbe de proximité objet P de l'ensemble optique constitué de cet oeil théorique et du composant optique suivant l'invention étant ainsi déterminée, un programme d'optimisation automatique, faisant appel notamment à une optimisation par la méthode des moindres carrés amortis, permet, ainsi qu'il est bien connu de l'homme de l'art, de déterminer la valeur des paramètres numériques à retenir.

Au lieu d'être bi-convexe, l'implant intra-oculaire 10 suivant l'invention peut tout aussi bien être par exemple convexe-plan, sa face arrière 18 ayant un rayon R2 suffisamment grand pour qu'elle puisse alors être considérée comme plane.

Il peut aussi être tout aussi bien plan-convexe, le rayon R1 de la sphère de base de la partie centrale asphérique 22 de sa face avant 17 étant alors lui-même relativement élevé.

Par ailleurs, au lieu d'être implanté dans la chambre antérieure 16 de l'oeil 11 à équiper, l'implant intra-oculaire 10 suivant l'invention peut tout aussi bien être implanté dans sa chambre postérieure.

En outre, suivant une deuxième forme de mise en oeuvre possible de l'invention, les paramètres numériques à déterminer peuvent être choisis de manière telle que, en vision de loin, l'aberration sphérique longitudinale due à l'ensemble optique constitué de l'oeil théorique pris en considération, cristallin en moins lorsque, comme en l'espèce, le composant optique concerné est un implant intra-oculaire 10, et de ce composant optique, est globalement corrigée.

Le tronçon TI de la courbe de proximité objet P correspondante est alors globalement vertical.

Enfin, au lieu d'être un implant intra-oculaire, le composant optique suivant l'invention peut tout aussi bien être une lentille de contact.

Dans ce cas, le cristallin de l'oeil théorique pris en considération est bien entendu conservé.

Dans les tableaux T1 et T2 suivants sont donnés, en référence aux figures 6 à 18, les paramètres numériques de divers composants optiques suivant l'invention, avec la nature de ceux-ci et leur mode d'implantation, ce mode d'implantation étant repéré par le sigle CA lorsqu'il s'agit de la chambre antérieure de l'oeil, et par le sigle CP lorsqu'il s'agit de sa chambre postérieure.

TABLEAU T1

| | Implan-tation | R1 | K | A2 $\times 10^{-2}$ | A3 $\times 10^{-3}$ | A4 $\times 10^{-4}$ | A5 $\times 10^{-5}$ | Figure |
|---|---|---|---|---|---|---|---|---|
| Exemple 1 Implant bi-convexe | CA | 13,450 | -132,085 | -0,1734521 | 0,6588392 | -0,7577453 | 0,3000454 | 6 |
| Exemple 2 Implant convexe-plan | CA | 6,095 | -14,103 | -0,0003297 | 0,8309988 | -1,141028 | 0,5263252 | 7 |
| Exemple 3 Implant plan-convexe | CA | 40,000 | -3002,10 | -0,4500209 | 0,8996099 | -0,9091179 | 0,3379623 | 8 |
| Exemple 4 Implant bi-convexe | CP | 12,056 | -140,977 | -0,1222130 | 0,5850626 | -0,6676928 | 0,2301316 | 9 |
| Exemple 5 Implant convexe-plan | CP | 5,526 | -14,4675 | 0,0812567 | 0,9338111 | -1,468438 | 0,7497366 | 10 |
| Exemple 6 Implant plan-convexe | CP | 30,000 | -1498,67 | -0,5268837 | 1,219160 | -1,408936 | 0,6006825 | 11 |

EP 0 398 813 B1

| | | | | | | | |
|---|---|---|---|---|---|---|---|---|
| **Exemple 7**<br>Implant<br>bi-convexe | CA | 13,418 | −145,681 | −0,1241714 | 0,5080515 | −0,6844913 | 0,3231505 | 12 |
| **Exemple 8**<br>Implant<br>convexe-plan | CA | 6,106 | −12,8283 | −0,0133428 | 0,7439685 | −1,067136 | 0,5192035 | 13 |
| **Exemple 9**<br>Implant<br>plan-convexe | CA | 40,000 | −3921,44 | −0,3759112 | 0,5819191 | −0,5171569 | 0,1652432 | 14 |
| **Exemple 10**<br>Implant<br>bi-convexe | CP | 12,149 | −132,152 | −0,1261559 | 0,5283329 | −0,7105776 | 0,3258733 | 15 |
| **Exemple 11**<br>Implant<br>convexe-plan | CP | 5,548 | −12,5946 | 0,0382755 | 0,8435873 | −1,344659 | 0,7089764 | 16 |
| **Exemple 12**<br>Implant<br>plan-convexe | CP | 40,000 | −3722,72 | −0,5627028 | 1,135583 | −1,357986 | 0,6273719 | 17 |
| **Exemple 13**<br>Lentille de<br>contact | ✕ | 7,24 | −6,90497 | 0,0355294 | 0,2450409 | −0,2881221 | 0,1164780 | 18 |

EP 0 398 813 B1

TABLEAU T2

| | Implantation | R2 | N1 | E1 | R3 | X3 | Y3 | Figure |
|---|---|---|---|---|---|---|---|---|
| **Exemple 1**<br>Implant bi-convexe | CA | -14 | 1,4920 | 0,78 | 32,7362 | 32,7130 | -0,8122 | 6 |
| **Exemple 2**<br>Implant convexe plan | CA | 40 | 1,4920 | 0,79 | 7,4785 | 7,4844 | -0,0287 | 7 |
| **Exemple 3**<br>Implant plan-convexe | CA | -8,047 | 1,4920 | 0,78 | -20,4506 | -20,4528 | 1,4649 | 8 |
| **Exemple 4**<br>Implant bi-convexe | CP | -12,5 | 1,4920 | 0,78 | 45,3353 | 45,2308 | -2,3653 | 9 |
| **Exemple 5**<br>Implant convexe-plan | CP | 40 | 1,4920 | 0,80 | 6,7762 | 6,7850 | -0,0131 | 10 |
| **Exemple 6**<br>Implant plan-convexe | CP | -7,555 | 1,4920 | 0,78 | -17,0687 | -17,0671 | 1,6757 | 11 |
| **Exemple 7**<br>Implant bi-convexe | CA | -14 | 1,4920 | 0,77 | 75,7360 | 75,6005 | -3,9158 | 12 |

EP 0 398 813 B1

| | | | | | | | | |
|---|---|---|---|---|---|---|---|---|
| Exemple 8 Implant convexe-plan | CA | 40 | 1,4920 | 0,77 | 8,5249 | 8,5068 | -0,2525 | 13 |
| Exemple 9 Implant plan-convexe | CA | -8,028 | 1,4920 | 0,76 | -15,2978 | -15,3005 | 1,4775 | 14 |
| Exemple 10 Implant bi-convexe | CP | -12,5 | 1,4920 | 0,77 | 124,6802 | 124,3391 | -8,5209 | 15 |
| Exemple 11 Implant convexe-plan | CP | 40 | 1,4920 | 0,78 | 7,6571 | 7,6465 | -0,1999 | 16 |
| Exemple 12 Implant plan-convexe | CP | -7,095 | 1,4920 | 0,77 | -11,3372 | -11,3455 | 1,4363 | 17 |
| Exemple 13 Lentille de contact | ✕ | 7,7200 | 1,377 | 0,22 | ✕ | ✕ | ✕ | 18 |

Bien entendu, la présente invention ne se limite pas aux exemples ainsi donnés, mais s'étend à toute variante dans les limites des revendications.

10

**Revendications**

1. Composant optique propre à la correction de la vision d'un individu, du genre comportant deux faces, l'une avant (17), l'autre arrière (18), dont l'une au moins forme, dans sa partie centrale au moins, une surface asphérique de révolution (22) de méridien répondant à la formule suivante :

$$x = \frac{1}{R_1}\left[\frac{Y^2}{1 +\sqrt{1 - (1+K)\ Y^2/R_1^2}}\right] + A2\ Y^4 + A3\ Y^6 + A4\ Y^8 + A5\ Y^{10}$$

dans laquelle R1, K, et A2, A3, A4 et A5 sont des paramètres numériques, caractérisé en ce que lesdits paramètres numériques sont choisis de manière telle que, pour l'ensemble optique constitué, d'une part, d'un oeil théorique donné dont on a enlevé le cristallin si le composant optique (10) concerné est un implant intra-oculaire, et, d'autre part, dudit composant optique, ils conduisent, pour la proximité objet P, définie par la formule :

$$P = N' \cdot \frac{dx'}{f'^2}$$

dans laquelle N' est l'indice du milieu image, dx' l'aberration sphérique longitudinale dans l'espace image, et f' la focale de l'oeil théorique choisi, à une courbe représentative,

   qui, (I) pour les valeurs fortes de la hauteur H des rayons lumineux par rapport à l'axe, comporte un premier tronçon (TI) sensiblement droit de pente au plus égale à zéro et tout entier situé entre une droite verticale (G1) passant par une origine de référence (O) quelconque donnée et une droite oblique (G2) passant par des points P1, P2 de coordonnées (-1 ; 1,5) et (-1,5 ; 2,75) par rapport à ladite origine de référence (O),

   qui, (II), pour les valeurs faibles de la hauteur H des rayons lumineux par rapport à l'axe, comporte un deuxième tronçon (TII) recoupant verticalement l'axe des dioptries entre des points P3, P4 de cet axe d'abscisses (-2,5) et (-4) par rapport à l'origine de référence (O),

   et qui, (III), pour les valeurs moyennes de la hauteur H des rayons lumineux par rapport à l'axe, comporte un tronçon médian (TIII), se raccordant de manière monotone et continue aux deux tronçons (TI, TII) précédents.

2. Composant optique suivant la revendication 1, caractérisé en ce que lesdits paramètres numériques sont choisis de manière telle que, en vision de loin, l'aberration sphérique longitudinale due à l'ensemble optique constitué de l'oeil théorique pris en considération, éventuellement cristallin en moins, et du composant optique concerné, soit sensiblement la même que celle due au seul dit oeil théorique, avec son cristallin.

3. Composant optique suivant la revendication 1, caractérisé en ce que lesdits paramètres numériques sont choisis de manière telle que, en vision de loin, l'aberration sphérique longitudinale due à l'ensemble optique constitué de l'oeil théorique pris en considération, éventuellement cristallin en moins, et du composant optique concerné, soit globalement corrigée.

4. Composant optique suivant l'une quelconque des revendication 1 à 3, caractérisé en ce que l'oeil théorique pris en considération présente les caractéristiques suivantes, parmi lesquelles les caractéristiques dimensionnelles sont exprimées en mm et K se rapporte à l'asphéricité des faces en cause :
   - rayon R4 de la face avant de la cornée 12 : 7,72 avec K = -0,26
   - rayon R5 de la face arrière de la cornée 12 : 6,5
   - épaisseur E2 de la cornée 12 : 0,55
   - distance D1 de la face arrière de la cornée 12 à la face avant du cristallin 25 : 3,05
   - rayon R6 de la face avant du cristallin 25 : 10,2 avec K = -3,1316
   - rayon R7 de la face arrière du cristallin 25 : -6 avec K = -1
   - épaisseur E3 du cristallin 25 : 4
   - distance D2 de la face arrière du cristallin 25 à la rétine 15 : 16,341
   - rayon R8 de la rétine 15 : -12
   - indice N2 de la cornée 12 : 1,367
   - indice N3 de l'humeur entre la cornée 12 et le cristallin 25 : 1,337
   - indice N4 du cristallin 25 : 1,42

- indice N5 de l'humeur entre le cristallin 25 et la rétine 15 : 1,336.

5. Composant optique suivant l'une quelconque des revendications 1 à 4, caractérisé en ce que la partie périphérique de la face (17) présentant une surface asphérique (22) dans sa zone centrale comporte une surface torique (23) dont le rayon de courbure est égal à celui de ladite surface asphérique (22) à son raccordement à celle-ci.

6. Composant optique suivant l'une quelconque des revendications 1 à 5, caractérisé en ce que l'étendue de la surface asphérique (22) est limitée à une circonférence de rayon au plus égal à 2,35 mm.

7. Composant optique suivant l'une quelconque des revendications 1 à 6, caractérisé en ce que l'indice N' du milieu image est égal à 1,336.

8. Composant optique suivant l'une quelconque des revendications 1 à 7, caractérisé en ce que la focale f' de l'oeil théorique choisi est supposée constante, en étant par exemple comprise entre 18 et 25 mm, et en étant par exemple choisie arbitrairement égale à 21,5 mm.

9. Composant optique suivant l'une quelconque des revendications 1 à 8, caractérisé en ce qu'il s'agit d'un implant intra-oculaire.

10. Composant optique suivant l'une quelconque des revendications 1 à 8, caractérisé en ce qu'il s'agit d'une lentille de contact.


## Patentansprüche

1. Optische Komponente zur Sehkorrektur einer Person, der Art, daß sie zwei Flächen aufweist, eine vordere (17), eine hintere (18), von denen zumindest eine zumindest in ihrem Mittelteil eine torische Meridian-Umdrehungs-Fläche (22) nach der folgenden Formel bildet:

$$x = \frac{1}{R_1}\left[\frac{Y^2}{1 + \sqrt{1 - (1+K)\ Y^2/R_1^2}}\right] + A2\ Y^4 + A3\ Y^6 + A4\ Y^8 + A5\ Y^{10}$$

bei der R1, K, A2, A3, A4 und A5 numerische Parameter sind, dadurch gekennzeichnet, daß die numerischen Parameter derart gewählt sind, daß sie für die optische Gesamtheit, die einerseits von dem gegebenen theoretischen Auge, dem man die Linse entnommen hat, falls die optische Komponente (10) ein intra-okulares Implantat ist, und andererseits von der optischen Komponente gebildet wird, für die Nähe Gegenstand P, definiert durch die Formel:

$$P = N' \cdot \frac{dx'}{f'^2}$$

bei der N' der Index des Bildbereichs, dx' die sphärische Längs-Aberration im Bildraum, und f' die Brennweite des ausgewählten theoretischen Auges ist, zu einer repräsentativen Kurve führen, die, (I) für die hohen Werte der Höhe H von Lichtstrahlen bezüglich der Achse einen ersten Abschnitt (TI) aufweist, der praktisch geradlinig eine Neigung von höchstens gleich Null hat, und gänzlich zwischen einer vertikalen Geraden (G1), die durch einen beliebig gegebenen Bezugspunkt (O) läuft, und einer schrägen Geraden (G2), die durch die Punkte P1, P2 mit den Koordinaten (-1; 1,5) und (-1,5; 2,75) bezüglich des Bezugspunktes (O) läuft, gelegen ist, die, (II) für die niedrigen Werte der Höhe H der Lichtstrahlen bezüglich der Achse einen zweiten Abschnitt (TII) aufweist, der vertikal die Dioptrie-Achse zwischen den Punkten P3, P4 dieser Abszissenachse (-2,5) und (-4) bezüglich des Bezugspunktes (O) schneidet, und die, (III) für die mittleren Werte der Höhe H der Lichtstrahlen bezüglich der Achse einen Mittelabschnitt (TIII) aufweist, der sich in monotoner und stetiger Weise an die zwei vorherigen Abschnitte (TI, TII) anschließt.

2. Optische Komponente gemäß Anspruch 1, dadurch gekennzeichnet, daß die numerischen Parameter derart gewählt sind, daß bei der Weitsicht die sphärische Längs-Aberration, die zu der optischen Gesamtheit gehört, die von dem in Erwägung gezogenen theoretischen Auge, eventuell zumindest einer Linse, und von der betreffenden optischen Komponente gebildet wird, praktisch gleich der ist, die zum theoretischen Auge allein mit seiner Linse gehört.

3. Optische Komponente gemäß Anspruch 1, dadurch gekennzeichnet, daß die numerischen Parameter derart gewählt sind, daß bei der Weitsicht die sphärische Längs- Aberration, die zu der optischen Gesamtheit gehört, die von dem in Erwägung gezogenen theoretischen Auge, eventuell zumindest einer Linse, und von der betreffenden optischen Komponente gebildet wird, global korrigiert ist.

4. Optische Komponente gemäß irgendeinem der Ansprüche 1 bis 3, dadurch gekennzeichnet, daß das in Erwägung gezogene theoretische Auge die folgenden Merkmale aufweist, unter denen die Maßmerkmale in mm ausgedrückt sind und sich K auf die torische Qualität der in Rede stehenden Flächen bezieht:
   - Radius R4 der vorderen Seite der Hornhaut 12: 7,72 mit K = -0,26
   - Radius R5 der hinteren Seite der Hornhaut 12: 6,5
   - Dicke E2 der Hornhaut 12: 0,55
   - Abstand D1 der hinteren Seite der Hornhaut 12 zur vorderen Seite der Linse 25: 3,05
   - Radius R6 der vorderen Seite der Linse 25: 10,2 mit K = -3,1316
   - Radius R7 der hinteren Seite der Linse 25: -6 mit K = 1
   - Dicke E3 der Linse 25: 4
   - Abstand D2 der hinteren Seite der Linse 25 zur Netzhaut 15: 16,341
   - Radius R8 der Netzhaut 15: -12
   - Index N2 der Hornhaut 12: 1,367
   - Index N3 des Kammerwassers zwischen der Hornhaut 12 und der Linse 25: 1,337
   - Index N4 der Linse 25: 1,42
   - Index N5 des Kammerwassers zwischen der Linse 25 und der Netzhaut 15: 1,336.

5. Optische Komponente gemäß irgendeinem der Ansprüche 1 bis 4, dadurch gekennzeichnet, daß der Randteil der Fläche (17), die eine torische Fläche (22) in ihrem Mittelbereich aufweist, eine torische Fläche (23) aufweist, deren Biegeradius gleich dem der torischen Fläche (22) bei ihrem Anschluß an diese ist.

6. Optische Komponente gemäß irgendeinem der Ansprüche 1 bis 5, dadurch gekennzeichnet, daß die Ausdehnung der torischen Fläche (22) auf einen Radiusumfang von höchstens gleich 2,35 mm begrenzt ist.

7. Optische Komponente gemäß irgendeinem der Ansprüche 1 bis 6, dadurch gekennzeichnet, daß der Index N' des Bildbereiches gleich 1,336 ist.

8. Optische Komponente gemäß irgendeinem der Ansprüche 1 bis 7, dadurch gekennzeichnet, daß die Brennweite f' des gewählten theoretischen Auges als konstant angenommen ist, wobei sie z.B. zwischen 18 und 25 mm liegt und wobei sie z.B. willkürlich gleich 21,5 mm gewählt ist.

9. Optische Komponente gemäß irgendeinem der Ansprüche 1 bis 8, dadurch gekennzeichnet, daß es sich um ein intra-okulares Implantat handelt.

10. Optische Komponente gemäß irgendeinem der Ansprüche 1 bis 8, dadurch gekennzeichnet, daß es sich um eine Kontaktlinse handelt.

## Claims

1. An optical component for correcting the vision of a person, of the type comprising two faces, a front face (17) and a rear face (18) of which one at least forms, in its central portion at least, an aspherical meridian surface (22) of revolution corresponding to the following formula:

$$x = \frac{1}{R_1}\left[\frac{Y^2}{1+\sqrt{1-(1+K)\ Y^2/R_1^2}}\right] + A2\ Y^4 + A3\ Y^6 + A4\ Y^8 + A5\ Y^{10}$$

in which R1, K and A2, A3, A4 and A5 are numerical paramaters, characterised in that said numerical parameters are so selected that, for the optical assembly formed on the one hand by a given theoretical eye from which the crystalline lens has been removed if the optical component (10) in question is an intra-

13

ocular implant and on the other hand said optical component, they result, for the object proximity P defined by the formula:

$$P = N' \cdot \frac{dx'}{f'^2}$$

in which N' is the index of the image medium, dx' is the longitudinal spherical aberration in the image space and f' is the focal length of the theoretical eye selected, in a representative curve

which, (I), for high values of the height H of the light rays relative to the axis, comprises a first substantially straight portion (T1) of a slope at most equal to zero and entirely disposed between a vertical straight line (G1) passing through any given reference origin (O) and an inclined straight line (G2) passing through points P1, P2 of co-ordinates (-1; 1.5) and (-1.5; 2.75) with respect to said reference origin (O),

which, (II), for low values of the height H of the light rays relative to the axis, comprises a second portion (TII) which vertically cuts the axis of the dioptres between points P3, P4 of said abscissa axis (-2.5) and (-4) with respect to the reference origin (O) and

which, (III), for medium values of the height H of the light rays relative to the axis, comprises a median portion (TIII) which connects monotonically and continuously to the two preceding portions (TI, TII).

2.  An optical component according to claim 1 characterised in that said numerical paramaters are so selected that, in far vision, longitudinal spherical aberration due to the optical assembly formed by the theoretical eye considered, possibly less the crystalline lens, and the optical component involved, is substantially the same as that due just to said theoretical eye with its crystalline lens.

3.  An optical component according to claim 1 characterised in that said numerical parameters are so selected that, in far vision, longitudinal spherical aberration due to the optical assembly formed by the theoretical eye considered, possibly less the crystalline lens, and the optical component involved, is totally corrected.

4.  An optical component according to any one of claims 1 to 3 characterised in that the theoretical eye in question has the following characteristics, of which the dimensional characteristics are expressed in mm and K relates to the asphericity of the faces referred to:
    - radius R4 of the front face of the cornea 12: 7.72 with K = -0.26
    - radius R5 of the rear face of the cornea 12: 6.5
    - thickness E2 of the cornea 12: 0.55
    - distance D1 of the rear face of the cornea 12 to the front face of the crystalline lens 25: 3.05
    - radius R6 of the front face of the crystalline lens 25: 10.2 with K = -3.1316
    - radius R7 of the rear face of the crystalline lens 25: -6 with K = -1
    - thickness E3 of the crystalline lens 25: 4
    - distance D2 of the rear face of the crystalline lens 25 to the retina 15: 16.341
    - radius R8 of the retina 15: -12
    - index N2 of the cornea 12: 1.367
    - index N3 of the humour between the cornea 12 and the crystalline lens 25: 1.337
    - index N4 of the crystalline lens 25: 1.42 and
    - index N5 of the humour between the crystalline lens 25 and the retina 15: 1.336.

5.  An optical component to any one of claims 1 to 4 characterised in that the peripheral portion of the face (17) having an aspherical surface (22) in its central region comprises a toric surface (23) of which the radius of curvature is equal to that of said aspherical surface (22) at its connection to same.

6.  An optical component according to any one of claims 1 to 5 characterised in that the extent of the aspherical surface (22) is limited to a circumference of a radius at most equal to 2.35 mm.

7.  An optical component according to any one of claims 1 to 6 characterised in that the index N' of the image medium is equal to 1.336.

8.  An optical component according to any one of claims 1 to 7 characterised in that the focal length f' of the theoretical eye selected is assumed to be constant, being for example between 18 and 25 mm and being for example arbitrarily selected equal to 21.5 mm.

9.  An optical component according to any one of claims 1 to 8 characterised in that it is an intra-ocular implant.

10. An optical component according to any one of claims 1 to 8 characterised in that it is a contact lens.

FIG.1

FIG.2

FIG.3

FIG.4

FIG.5

## FIG 6

## FIG 7

## FIG 8

## FIG 9

## FIG 10

## FIG 11

FIG 12

FIG 13

FIG 14

## FIG 15

## FIG 16

## FIG 17

## FIG 18